(19) Europäisches Patentamt
European Patent Office
Office européen des brevets

(11) **EP 3 280 495 B1**

(12) **EUROPEAN PATENT SPECIFICATION**

(45) Date of publication and mention
of the grant of the patent:
**11.09.2024   Bulletin 2024/37**

(21) Application number: **16719993.4**

(22) Date of filing: **08.04.2016**

(51) International Patent Classification (IPC):
**A61N 7/02** *(2006.01)*      **A61N 7/00** *(2006.01)*
**A61B 18/00** *(2006.01)*      **A61B 90/00** *(2016.01)*
**A61B 34/20** *(2016.01)*      **A61B 17/00** *(2006.01)*

(52) Cooperative Patent Classification (CPC):
**A61N 7/00; A61N 7/02;** A61B 2017/00039;
A61B 2018/00904; A61B 2034/2048;
A61B 2034/2055; A61B 2034/2059;
A61B 2090/065; A61B 2560/0406; A61B 2560/0418

(86) International application number:
**PCT/US2016/026794**

(87) International publication number:
**WO 2016/164829 (13.10.2016 Gazette 2016/41)**

(54) **SYSTEM AND METHOD FOR INCREASED CONTROL OF ULTRASOUND TREATMENT**

SYSTEM ZUR ERHÖHTEN STEUERUNG EINER ULTRASCHALLBEHANDLUNG

SYSTÈME POUR UN RÉGLAGE AMÉLIORÉ D'UN TRAITEMENT PAR ULTRASONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **08.04.2015   US 201562144647 P**

(43) Date of publication of application:
**14.02.2018   Bulletin 2018/07**

(60) Divisional application:
**24190576.9**

(73) Proprietor: **Guided Therapy Systems, LLC**
**Scottsdale, AZ 85251 (US)**

(72) Inventors:
• **BARTHE, Peter G.**
  **deceased (US)**
• **SLAYTON, Michael H.**
  **Phoenix, AZ 85018 (US)**

(74) Representative: **Maiwald GmbH**
**Elisenhof**
**Elisenstraße 3**
**80335 München (DE)**

(56) References cited:
US-A1- 2006 122 509      US-A1- 2009 163 807
US-A1- 2012 165 668      US-A1- 2012 165 668
US-A1- 2013 012 755      US-A1- 2015 088 182
US-A1- 2015 088 182      US-A1- 2015 088 182

EP 3 280 495 B1

## Description

### BACKGROUND

Existing ultrasound treatment systems deploy ultrasound probes that have physically bulky hardware. These probes have large patient contact footprints and limit operator control of the delivery of ultrasound energy to the patient.

Handheld probes generally take the form of an elongated handle for holding and positioning the probe. The probes have an emission portion that is physically larger than the elongated handle in a dimension that is roughly normal to the direction of ultrasound transmission.

Existing ultrasound probes lack sufficient maneuverability to allow treatment to complex regions of the body that have three-dimensional contours and tight spaces, such as the area between fingers. Additionally, existing treatment devices are limited by fixed treatment patterns, such as lines, patches, or other large planar contours.

Accordingly, a need exists for systems for ultrasound treatment where the patient contact footprint is reduced and a user has increased control over the exact placement of energy. Prior art can be found in US 2012/165668, US 2015/088182 and US 2006/122509.

### SUMMARY

The present disclosure overcomes the aforementioned drawbacks by presenting systems for ultrasound treatment with a reduced patient contact footprint and improved ultrasound probe maneuverability.

In one aspect, this disclosure provides an ultrasound treatment system. The ultrasound treatment system can include an ultrasound probe, a controller, and a power supply. The ultrasound probe can include a contact surface. The ultrasound probe can be movable by a user along a treatment surface in an arbitrary surface pattern while maintaining contact between the contact surface and the treatment surface. The controller can be operably coupled to the ultrasound probe. The controller can, in use, control the emission of therapeutic ultrasound energy from the ultrasound probe. The power supply can, in use, provide power to the ultrasound probe sufficient for the emission of the therapeutic ultrasound energy. The ultrasound probe can transmit the therapeutic ultrasound energy into a treatment pattern beneath the treatment surface that mimics the arbitrary surface pattern.

In another aspect, this disclosure provides an ultrasound treatment system. The ultrasound treatment system can include an ultrasound probe, a power supply, and a controller. The ultrasound probe can have a transducer and a user interface. The transducer can, in use, emit a therapeutic ultrasound energy. The user interface can generate a first signal in response to a first user command and a second signal in response to a second user command. The power supply can, in use, provide power to the transducer sufficient for the emission of the therapeutic ultrasound energy. The controller can be operably coupled to the transducer and the user interface. The controller can, in use, control the emission of the therapeutic ultrasound energy from the transducer. The controller can, in use, receive signals from the user interface. The controller can, in response to the first signal, direct the transducer to emit a first therapeutic ultrasound energy. The controller can, in response to the second signal, direct the transducer to emit a second therapeutic ultrasound energy. The first therapeutic ultrasound energy and the second therapeutic ultrasound energy can have at least one different spatial or temporal property.

According to the invention, the ultrasound system includes an ultrasound probe, a controller, a position sensor, and a power supply. The ultrasound probe can have a contact surface. The ultrasound probe can be movable by a user along a treatment surface in an arbitrary surface pattern while maintaining contact between the contact surface and the treatment surface. The controller can be operably coupled to the ultrasound probe. The controller can, in use, control the emission of a therapeutic ultrasound energy from the ultrasound probe. The position sensor can, in use, measure a position of the ultrasound probe along the treatment surface and transmit a position signal to the controller corresponding to the position of the ultrasound probe along the treatment surface. The power supply can, in use, provide power to the ultrasound probe sufficient for the emission of the therapeutic ultrasound energy. The controller can, in response to the position signal indicating that the ultrasound probe is moving along the treatment surface, and in response to the measured position, vary a pulse width of the therapeutic ultrasound energy, a power amplitude of the therapeutic ultrasound energy, and a timing between pulse of the therapeutic ultrasound energy to produce a series of ultrasound pulse that have equal energy and are equally spaced along the surface pattern.

In a further aspect, this disclosure provide a non-claimed method of depositing therapeutic ultrasound energy from an ultrasound probe into a target medium. The method can include one or more of the following steps: a) coupling the ultrasound probe to the target medium; b) subsequent to step a), activating emission of therapeutic ultrasound energy from the ultrasound probe; c) subsequent to step b), moving the ultrasound probe along a treatment surface above the target medium in an arbitrary surface pattern, thereby directing therapeutic ultrasound energy from the ultrasound probe into the target medium in a treatment pattern that mimics the surface pattern; and d) subsequent to step c), deactivating emission of the therapeutic ultrasound energy from the ultrasound probe.

In yet another aspect, this disclosure provide a non-claimed method of depositing therapeutic ultrasound energy from an ultrasound probe into a target medium. The method can include one or more of the following steps: a) coupling the ultrasound probe to the target medium; b) subsequent to step a), moving the ultrasound probe along a treatment surface above the target medium in an arbitrary surface pattern; c) activating a user interface of the ultrasound probe for either less than a pre-determined length of time or greater than or equal to the pre-determined length of time when the ultrasound probe is at a first location; d) if the user interface is activated for less than the pre-determined length of time, directing a single therapeutic ultrasound pulse into the target medium beneath the surface pattern at the first location; e) if the user interface is activated for greater than or equal to the pre-determined length of time, directing two or more therapeutic ultrasound pulses into the target medium beneath the surface pattern, the first of the two or more therapeutic ultrasound pulses directed into the target medium at the first location and the second of the two or more therapeutic ultrasound pulses directed into the target medium at a distance from the location along the surface pattern.

In another aspect, this disclosure provide a non-claimed method of depositing therapeutic ultrasound energy from an ultrasound probe into a target medium. The method can include one or more of the following steps: a) coupling the ultrasound probe to the target medium; b) subsequent to step a), activating emission of therapeutic ultrasound energy from the ultrasound probe; c) subsequent to step b), moving the ultrasound probe along a treatment surface above the target medium in an arbitrary surface pattern, thereby directing therapeutic ultrasound energy from the ultrasound probe into the target medium in a treatment pattern that mimics the surface pattern; and d) subsequent to step c), deactivating emission of the therapeutic ultrasound energy from the ultrasound probe.

The foregoing and other aspects and advantages of the invention will appear from the following description. In the description, reference is made to the accompanying drawings which form a part hereof, and in which there is shown by way of illustration a preferred aspect of the disclosure. Such aspect does not necessarily represent the full scope of the disclosure, however, and reference is made therefore to the claims and herein for interpreting the scope of the disclosure.

BRIEF DESCRIPTION OF THE DRAWINGS

Fig. 1 is an illustration of an ultrasound probe, according to one aspect of the present disclosure.
Fig. 2 is an ultrasound system, according to one aspect of the present disclosure.
Fig. 3 is an illustration of an ultrasound probe executing an arbitrary treatment pattern, according to one aspect of the present disclosure.
Fig. 4 is an illustration of an ultrasound probe executing a circular treatment pattern, according to one aspect of the present disclosure.
Fig. 5 is an illustration of an ultrasound probe applying a treatment pattern to the area adjacent to an eye of a patient, according to one aspect of the present disclosure.
Fig. 6 is an image of linear, arbitrary, and circular treatment patterns generated by an ultrasound probe, according to one aspect of the present disclosure.
Fig. 7 is a flowchart showing a method, according to one aspect of the present disclosure.
Fig. 8 is a flowchart showing a method, according to one aspect of the present disclosure.
Fig. 9 is a flowchart showing a method, according to one aspect of the present disclosure.
Fig. 10 is a Schlieren image of an ultrasound emission from an ultrasound probe, according to one aspect of the present disclosure.
Fig. 11 is a Schlieren image of an ultrasound emission from an ultrasound probe, according to one aspect of the present disclosure.
Fig. 12 is a photograph of a treatment device, according to one aspect of the present disclosure.
Fig. 13 is a photograph of the gross pathology of ultrasound lesions generated by an ultrasound probe in Example 1, according to one aspect of the present disclosure.
Fig 14 is a photograph of the gross pathology of ultrasound lesions generated by an ultrasound probe in Example 2, according to one aspect of the present disclosure.

DETAILED DESCRIPTION

Before the present invention is described in further detail, it is to be understood that the invention is not limited to the particular embodiments described. It is also to be understood that the terminology used herein is for the purpose of describing particular embodiments only, and is not intended to be limiting. The scope of the present invention will be limited only by the claims. As used herein, the singular forms "a", "an", and "the" include plural embodiments unless the context clearly dictates otherwise. Where a range of values is recited, this disclosure contemplates all combinations of the upper and lower bounds of those ranges that are not explicitly disclosed. For example, if ranges of 1 to 10 and 2 to 9 are disclosed, this disclosure contemplates ranges of 1 to 9 and 2 to 10. Aspects of the present disclosure that are referenced with respect to systems are applicable to the disclosed methods, and vice versa, unless the context clearly dictates otherwise. Similarly, aspects of the present disclosure that are referenced with respect to one exemplary system are applicable to other disclosed systems or with respect to one method are applicable to the other disclosed methods, unless the context clearly dictates otherwise.

Specific structures, devices, and methods relating to improved ultrasound treatment efficiency and operation are disclosed. Methods do not form part of the claimed invention, which is defined by the independent claim. It should be apparent to those skilled in the art that many additional modifications beside those already described are possible without departing from the inventive concepts. In interpreting this disclosure, all terms should be interpreted in the broadest possible manner consistent with the context. Variations of the term "comprising" should be interpreted as referring to elements, components, or steps in a non-exclusive manner, so the referenced elements, components, or steps may be combined with other elements, components, or steps that are not expressly referenced. Embodiments referenced as "comprising" certain elements are also contemplated as "consisting essentially of" and "consisting of" those elements.

As used herein, "maximum physical dimension" shall refer to the largest measurable size of an object or geometric shape. For example, the maximum physical dimension of a circle is the circle's diameter, the maximum physical dimension of a square or rectangle is the square or rectangle's diagonal, etc.

This disclosure provides systems for ultrasound treatment with a reduced patient contact footprint and improved ultrasound probe maneuverability. The reduced footprint and increased maneuverability can allow motion that can conform to regions of interest of the body which have complex three-dimensional contours and tight spaces, such as the areas between the fingers.

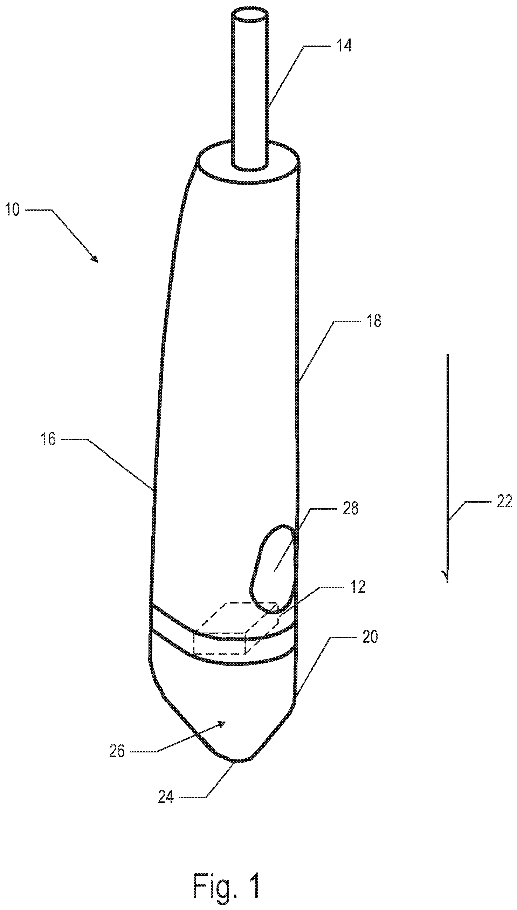

Referring to Fig. 1, this disclosure provides an ultrasound probe 10. The ultrasound probe 10 can include an ultrasound transducer 12 for emitting ultrasound energy. The ultrasound probe 10 can have a cable bundle 14 (as illustrated), or can have a wireless interface for communication with other aspects of the system or can be configured as a stand-alone system with all aspects that are necessary for function of the ultrasound probe 10 located within the probe itself. The ultrasound probe 10 can include a probe housing 16. The probe housing 16 can include a handle portion 18 and a tip portion 20.

The ultrasound transducer 12 can be a single transduction element or a multi-element array. The ultrasound transducer 12 can emit therapeutic ultrasound energy.

The handle portion 18 can have a length along an axial direction 22 that is greater than a maximum diameter normal to the axial direction 22. In certain aspects, the handle portion 18 has a length along the axial direction 22 that is at least twice the maximum diameter normal to the axial direction 22, including but not limited to, a length that is at least three times the maximum diameter, at least four times the maximum diameter, at least five times the maximum diameter, or at least ten times the maximum diameter. The handle portion 18 can be configured to fit comfortably in the hand of an adult human. The handle portion 18 can have a smooth surface or can have indentations intended to accommodate the fingers of a user. The handle portion 18 can have a maximum physical dimension orthogonal to the axial direction 22 ranging from 3 mm to 50 mm, including but not limited to, a maximum physical dimension orthogonal to the axial direction 22 ranging from 5 mm to 25 mm. The handle portion 18 can have a diameter ranging from 3 mm to 50 mm, including but not limited to, a diameter ranging from 5 mm to 25 mm. The handle portion 18 can have a length along the axial direction 22 ranging from 10 mm to 200 mm, including but not limited to, a length along the axial direction ranging from 20 mm to 150 mm.

The tip portion 20 can include a contact surface 24 for coupling the ultrasound energy to a target surface. The contact surface 24 can have a flat shape, or a convex shape, or a rectangular concave or convex shape. In certain aspects, the tip portion 20 and contact surface 24 can be the same material. In certain aspects, the tip portion 20 and contact surface 24 can be composed of different materials. In certain aspects, the tip portion 20 has a thickness of approximately an integer multiple of one-half wavelength of the ultrasound energy. In certain aspects the tip portion 20 is acoustically transparent, such as an acoustically transparent thin film. The contact surface 24 can have a convex curved shape.

In certain aspects, the handle portion 18, the tip portion 20, or both can have one or more bends such that the handle portion 18, the tip portion 20, or both form a non-zero angle with respect to the axial direction 22. The tip portion 20 can have a tapered shoulder, such that its maximum physical dimension orthogonal to the axial direction 22 reduces as the tip portion 20 extends away from the handle portion 18 along the axial direction 22. The tip portion 20 can have a circular cross section such that its cross section normal to the axial direction 22 reduces as the tip portion 20 extends away from the handle portion 18 along the axial direction 22. The tip portion 20 can have a rectangular cross section such that its cross section normal to the axial direction 22 reduces as the tip portion 20 extends away from the handle portion 18 along the axial direction 22.

The tip portion 20 can have a maximum physical dimension orthogonal to the axial direction 22 ranging from 1 mm to 40 mm, including but not limited to, a maximum physical dimension orthogonal to the axial direction ranging from 3 mm to 25 mm, from 5 mm to 30 mm, from 10 mm to 20 mm, or from 2 mm to 35 mm. The tip portion 20 can have diameters or widths ranging from 1 mm to 40 mm, including but not limited to, diameters or widths ranging from 3 mm to 25 mm, from 5 mm to 30 mm, from 10 mm to 20 mm, or from 2 mm to 35 mm. The tip portion 20 can have a length along the axial direction 22 ranging from 3 mm to 50 mm, including but not limited to, a length along the axial direction 22 ranging from 5 mm to 25 mm, from 10 mm to 20 mm, from 15 mm to 30 mm, or from 25 mm to 40 mm.

In certain aspects, the ultrasound probe 10 can emit ultrasound in a direction that is substantially parallel to the axial direction 22. In certain aspects, the ultrasound probe 10 can emit ultrasound in direction that is not parallel to the axial direction 22. In certain aspects, the ultrasound probe 10 can emit ultrasound in more than one direction.

In certain aspects, the contact surface 24 can have a maximum physical dimension of less than 2.5 cm, less than 2 cm, less than 1 cm, less than 9 mm, less than 8 mm, less than 7 mm, less than 6 mm, less than 5 mm, less than 4 mm, or less than 3 mm. In certain aspects, the contact surface 24 can have a diameter or width of less than 2 cm, less than 1 cm, less than 9 mm, less than 8 mm, less than 7 mm, less than 6 mm, less than 5 mm, less than 4 mm, or less than 3 mm.

A strong acoustic focus can be characterized by an f-number, which equals the transducer focal depth divided by the transducer aperture. In certain aspects, the f-number can range from 0.7 to 1.5. In certain aspects, a deep treatment (i.e., a large focal depth) can entail a large ultrasound transducer 12 aperture and a correspondingly large tip portion 20. In certain aspects, a superficial treatment (i.e., a small focal depth) can entail a much smaller tip portion 20. In certain aspects the length of the tip portion 20 along the axial direction 22 is on the order of the focal depth of a transducer 12 that is focused. For example, if transducer 12 is focused 3 mm deep into tissue and transducer 12 has an acoustic focus of 15 mm, then tip portion 12 can have an axial length about 12 mm to place the focus at the desired position. In certain aspects the length of tip portion 20 along the axial direction 22 is less than 30 mm or less than 20 mm or less than 10 mm.

The transducer 12 can be positioned within the probe housing at a distance from the contact surface 24 that allows the nose portion 20 to be lengthened to allow visual feedback of the point of contact between the contact surface 24 and a treatment surface. In certain aspects, the transducer 12 can be positioned at a distance from the contact surface 24 that is at least equal to a focal depth of the ultrasound from the contact surface 24, including but not equal to, a distance from the contact surface 24 that is at least 125%, at least 150%, at least 200%, at least 300%, at least 400%, or at least 500% of a focal depth of the ultrasound from the contact surface 24.

In certain aspects, the tip portion 20 can be detachable from the handle portion 18. In certain aspects, the tip portion 20 can function as an acoustic waveguide. In certain aspects, the tip portion 20 can be swappable and/or disposable. The tip portion 20 can include the ultrasound transducer 12, so the tip portion 20 including the ultrasound transducer 12 can be detachable, swappable, and/or disposable. The tip portion, with or without the ultrasound transducer 12, can be single-use or limited-use. The usage of the tip portion 20, with or without the ultrasound transducer 12, can be monitored to ensure that the tip portion 20 is not used past a usable lifetime. One example of a system for monitoring the use lifetime of the tip portion 20 is a crypto chip.

An acoustic coupling medium 26 can occupy the volume between the ultrasound transducer 12 and the contact surface 24. In certain applications, the acoustic coupling medium 26 is incorporated into the tip portion 20 itself, as a liquid, gel, or a solid.

The ultrasound probe 10 can include a user interface 28, which can take the form of a button, a touchpad, a switch, or other forms that can be manipulated by a user to control the emission of ultrasound energy. The user interface 28 can be used to activate or deactivate the emission of ultrasound energy. The user interface 28 can be located on the handle portion 18. The user interface 28 can be located in a position where a user's finger or thumb naturally falls when holding the ultrasound probe 10. A remote user interface 34 can be positioned remote from the ultrasound probe 10 as described elsewhere herein.

The ultrasound probe 10 can deliver pulsed ultrasound energy having pulse widths ranging from 1 ns to 10 seconds per pulse, including but not limited to, pulse widths ranging from 1 $\mu$s to 100 ms, or from 100 $\mu$s to 50 ms. The ultrasound probe 10 can also emit continuous ultrasound energy.

The ultrasound probe 10 can deliver pulsed ultrasound energy having power amplitudes ranging from 1 mW to 10 kW per pulse, including but not limited to, power amplitudes ranging from 0.5 W to 1 kW per pulse, or 1 W to 200 W. The ultrasound probe 10 can deliver continuous ultrasound energy having power amplitudes in the same ranges as the pulsed ultrasound energy, including zero amplitude.

The ultrasound probe 10 can deliver pulsed ultrasound energy having periods ranging from 1 $\mu$s to 10 s, including but not limited to, periods ranging from 10 ms to 1 s, or from 100 ms to 0.5 s. The ultrasound probe 10 can deliver continuous ultrasound energy for a single defined length of time or can deliver continuous ultrasound energy that is emitted when a user activates the user interface 26.

The ultrasound transducer 12 can emit ultrasound energy at frequencies ranging from 500 kHz to 100 MHz, including but not limited to, frequencies ranging from 1 MHz to 20 MHz. In certain aspects, the ultrasound transducer 12 can be configured as a broadband emitter capable of delivering wideband pulses. In certain aspects, the ultrasound transducer 12 can be configured to operate at multiple distinct frequencies simultaneously.

The ultrasound transducer 12 can be controlled to deliver ultrasound energy with varying spatial and temporal characteristics. In some aspects, the spatial and temporal characteristics are varied by user programming. In some aspects, the spatial and temporal characteristics are varied as the result of feedback from one or more of the sensors described herein.

The ultrasound probe 10 can be configured to deliver focused ultrasound energy that focuses at distances ranging from 0.1 mm to 30 mm from the contact surface 24, including but not limited to, distances ranging from 0.2 mm to 10 mm, from 0.5 mm to 5 mm, from 1 mm to 4 mm, or from 2 mm to 3 mm from the contact surface 24.

In certain aspects, the ultrasound probe 10 can emit focused ultrasound energy. In certain aspects, the ultrasound probe 10 can emit unfocused ultrasound energy. In certain aspects, the ultrasound probe 10 can emit defocused ultrasound energy. Focusing, defocusing, or no focusing can be achieved via concave, convex, or flat eletroacoustic transduction elements. In certain aspects, beamshaping control can be achieved with lenses, electronic phasing, and arrays. Beamshaping elements can be disposed within the handle portion 18, the tip portion 20, the contact surface 24, or a combination thereof. The ultrasound transducer 12 with beamshaping element can be movable, exchangeable, or programmatically movable or exchangeable in response to feedback or labeling. The tip portion 20 or contact surface 24 including beamshaping elements can be movable, exchangeable, or programmatically movable or exchangeable in response to feedback or labeling.

In certain aspects, the ultrasound transducer 12 can be affixed to remain stationary within the probe housing 16. The ultrasound transducer 12 can be stationary relative to the contact surface 24. Certain existing technologies produce multiple pulses of ultrasound in distinct locations by moving a transducer within a housing by a motion mechanism, such as a motor. The present disclosure does not require this type of motion mechanism to achieve the disclosed effects. The ultrasound treatment pattern is thus controlled by a user by moving the ultrasound probe 10 along a treatment surface in a surface pattern that mimics the desired treatment pattern.

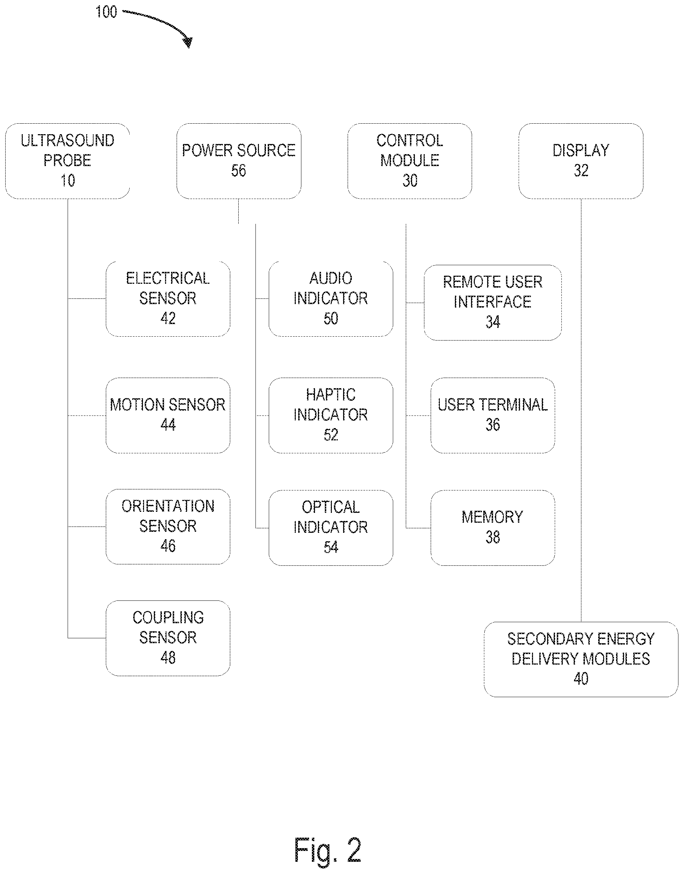

Referring to Fig. 2, this disclosure provides an ultrasound system 100. The ultrasound system 100 can include the ultrasound probe 10 as described herein, and one or more of the following features: a power source 56; a controller 30; a display 32; a remote user interface 34; a user terminal 36; a memory 38; one or more secondary energy delivery modules 40; an electrical sensor 42; a motion sensor 44; an orientation sensor 46; a coupling sensor 48; an audio indicator 50; a haptic indicator 52; and an optical indicator 54. These features can be located in or on the ultrasound probe 10 or remote from the ultrasound probe 10.

The power source 56 can be an electric power providing means known to those having ordinary skill in the art to be capable of powering an ultrasound system 100 as described herein. Examples of power sources 28 can include, but are not limited to, alternating current (AC) to direct current (DC) power supplies capable of providing electrical energy to the ultrasound probe 10 and other parts of the ultrasound system 100 that require power, a battery capable of generating at least 1 watt, and the like. The power source 56 can also include charging circuits for charging a rechargeable power supply, such as a battery. In some aspects, the power source 56 can generate power ranging from 1 watt to 100 watts. In addition, a radiofrequency driver can provide kHz or MHz drive frequencies to the ultrasound transducer 12.

The controller 30 can be a computing system or purely electronic system capable of providing instructions or control to the various electronic components of the ultrasound system 100 in order to operate the ultrasound system 100 as described herein. In certain aspects, a user interface 28 switch can activate energy directly. The controller 30 can direct the ultrasound transducer 12 to emit ultrasound energy having pre-determined spatial and temporal parameters.

The display 32 can be a visual means capable of communicating relevant information to a user. Examples of displays 32 can include, but are not limited to, computer monitors, a display screen on a personal device, such as a smart phone, a tablet, a smart watch, or the like, a projection system, and the like.

The remote user interface 34 can be an interface capable of manipulation by a user to control the emission of ultrasound energy, including treatment settings, such as power and timing. The remote user interface 34 can be used to activate or deactivate the emission of ultrasound energy. Examples of remote user interfaces 34 include, but are not limited to, a foot switch, a microphone equipped with voice recognition software, a camera equipped with gesture recognition software, and the like.

The memory 38 can be any storage medium capable of electronically storing parameters or other information relevant to the operation of the ultrasound system 100. Examples of memory 38 include, but are not limited to, EEPROM, hard drives, flash memory, and other similar means of electronic storage of information.

The user terminal 36 can be a remote system suitable for receiving user instruction relating to aspects of operation of the ultrasound system 100 that are not the activation or deactivation of the emission of the ultrasound energy. Examples of a user terminal 36 can include, but are not limited to, an input to computer, such as a keyboard or a mouse, an input on personal device, such as a touch screen on a smart phone, a tablet, a smart watch, or the like, a microphone equipped with voice recognition software, a camera equipped with gesture recognition software, a foot switch, and the like.

The one or more secondary energy delivery modules 40 can include a photon-based energy source, a radiofrequency energy source, a thermal energy source, or a mechanical energy source. The secondary energy delivery modules 40 can be located within the ultrasound probe 10 or as a separate module.

Electrical sensors 42 can be deployed to monitor the distribution and usage of electrical signals within the systems. Examples of electrical sensors 42 can include, but are not limited to, current sensors, voltage sensors, power sensors, and the like, and combinations thereof. The electrical sensors 42 can be located within the ultrasound probe 10 or in other portions of the ultrasound system 100 which require electrical signal monitoring.

Many sensors can function as both motion sensors 44 and orientation sensors 46, with the distinction lying in the type of information that is extracted from the sensor. A motion sensor 44 functions to determine motion or lack of motion of the ultrasound probe 10 relative to a pre-existing position. An orientation sensor 46 functions to determine a relative positioning of the ultrasound probe 10 relative to an external frame, such as a gravitational frame or a surface of a patient's skin. Examples of motion sensors 44 and orientation sensors 46 can include, but are not limited to, accelerometers, gyroscopes, magnetometers, geomagnetic sensors, global positioning systems, optical sensors, gesture sensors, a camera and associated motion or orientation detecting software, and other sensors capable of measuring the motion or orientation of the ultrasound probe 10.

A coupling sensor 48 determines if the ultrasound source is acoustically coupled to the target medium. Examples of coupling sensors 48 can include, but are not limited to, a capacitive sensor, a system that utilizes a frequency sweep function to determine coupling, such as the system disclosed in U.S. Patent Application No. 13/547,012, and other sensors capable of measuring whether the ultrasound probe 10 is coupled to its target. In certain aspects, the controller 30 can receive a signal from the coupling sensor 48, and can terminate emission of ultrasound energy in response to a signal indicating that the ultrasound probe 10 is not coupled to the target medium.

An audio indicator 50 provides an audio signal to a user. Examples of an audio indicator 50 include, but are not limited to, a speaker, and other similar audio generation devices. The audio indicator 50 can be located within the ultrasound probe 10 or external to the ultrasound probe 10.

A haptic indicator 52 provides a haptic signal to a user. Examples of a haptic indicator 52 include, but are not limited to, a vibratory motor, an electroactive polymer, a piezoelectric material, and other similar haptic generation devices. The haptic indicator 52 can be located within the ultrasound probe 10 or external to the ultrasound probe 10. If the haptic indicator 52 is external to the ultrasound probe 10, the haptic indicator 52 can be located in a wearable technology, such as a wrist band, in a form that a user can contact, such as a foot mat or a seat, or in a holdable form, such as a portable device.

An optical indicator 54 provides a visual signal to a user. The optical indicator 54 should be located in a position where the user can see the optical indicator 54 itself or the light that is emitted from the optical indicator 54. In one aspect, the optical indicator 54 can project light to the target surface so the user can monitor the target surface and receive the visual signal simultaneously. Examples of an optical indicator 54 include, but are not limited to, a light emitting diode (LED), a fiber optic coupled to a light source, and other similar light generating devices. The optical indicator 54 can be located within the ultrasound probe 10 or external to the ultrasound probe 10.

In certain aspects, transducer element temporal and/or spatial apodization is utilized to mitigate and control acoustic edge effects.

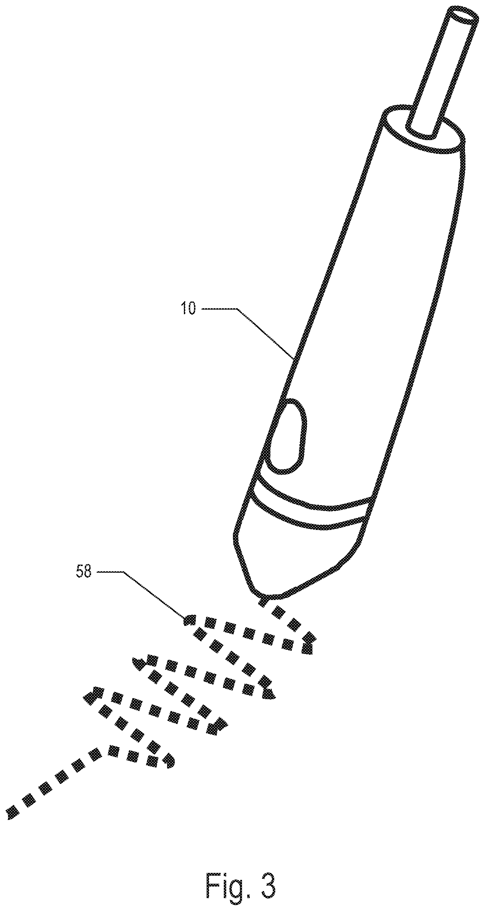

Referring to Fig. 3, an ultrasound probe 10 is illustrated moving along a target surface, the ultrasound probe 10 emitting controlled ultrasound energy to generate a treatment pattern 58 beneath the target surface. The treatment pattern 58 consists of lesions that are roughly equal in size and relative spacing. The treatment pattern 58 that is illustrated is an arbitrary treatment pattern (shown as a zig-zag or curvilinear pattern for the sake of illustration).

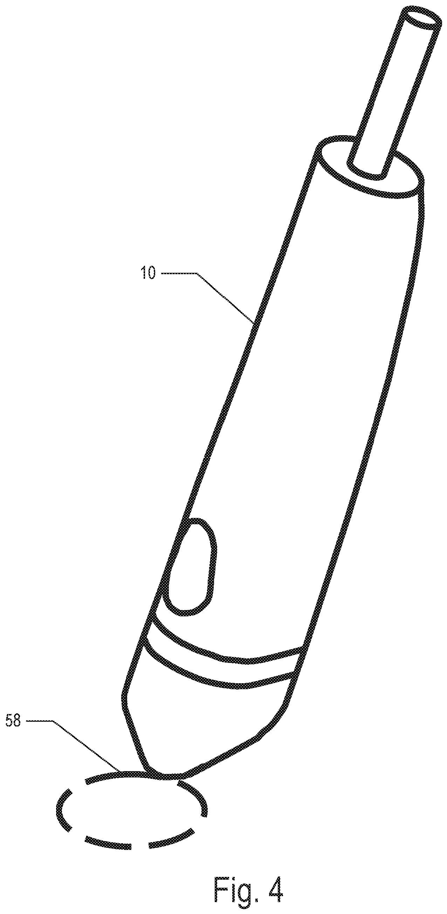

Referring to Fig. 4, an ultrasound probe 10 is illustrated moving along a target surface, the ultrasound probe 10 emitting controlled ultrasound energy to generate a treatment pattern 58 beneath the target surface. The treatment pattern consists of lesions that are roughly equal in relative spacing. The treatment pattern 58 that is illustrated is a circular pattern.

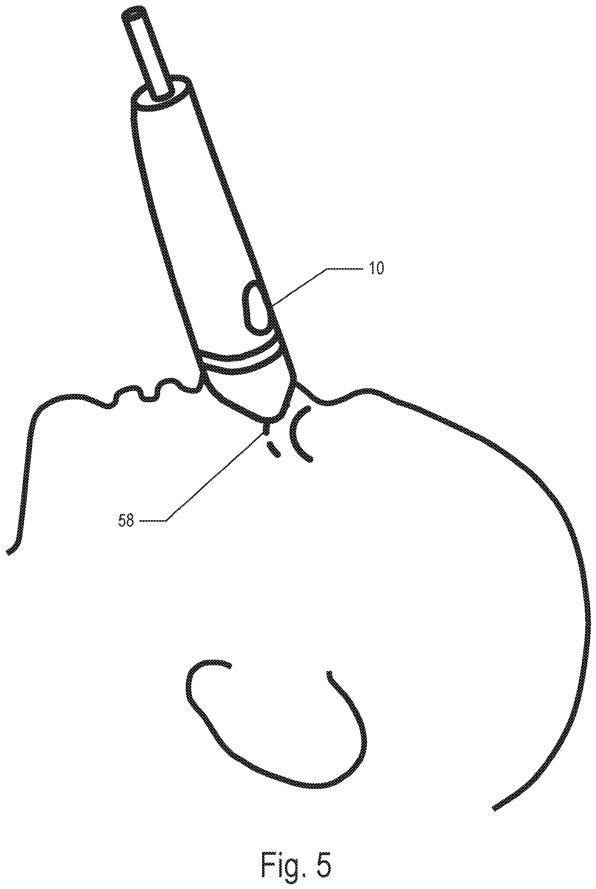

Referring to Fig. 5, an ultrasound probe 10 is coupled to a skin surface beneath a patients eye and moved along the skin surface, the ultrasound probe 10 emitting controlled ultrasound energy to generate a treatment pattern beneath the skin surface. The treatment pattern 58 consists of lesions that are roughly equal in size. The treatment pattern 58 that is illustrated is a curved pattern. In certain aspects, the treatment pattern 58 can be located along periorbital locations. In certain aspects, the treatment pattern 58 can be located along perioral locations. In certain aspects, the treatment pattern 58 can be located along perinasal locations. In certain aspects, the treatment pattern 58 can be located along tendons, ligaments, muscles, and other musculoskeletal tissue.

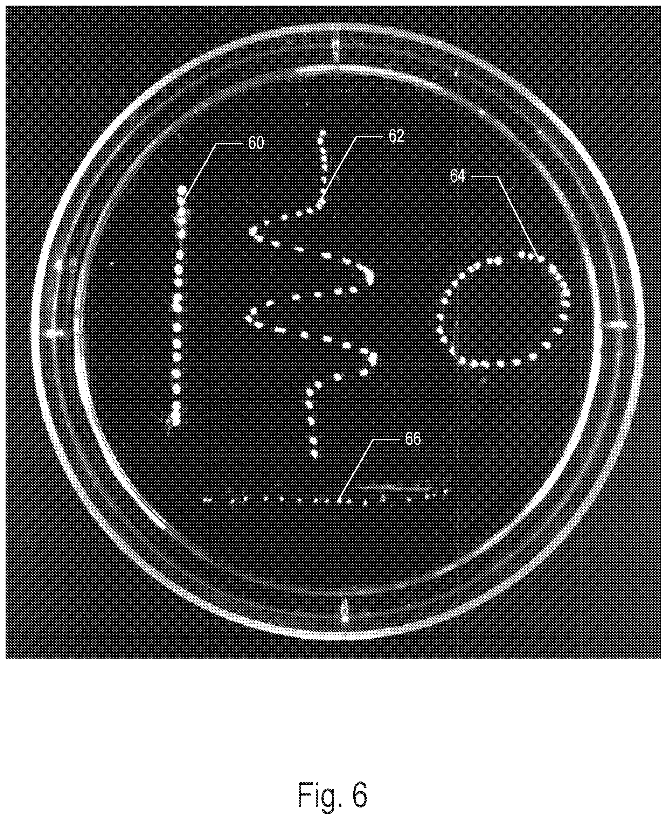

Referring to Fig. 6, four different treatment patterns 58 were generated in a plastic petri dish acoustically coupled to tip portion 20 with water. A high-intensity linear treatment pattern 60, a high-intensity zig-zag treatment pattern 62, a high-intensity circular treatment pattern 64, and a low-intensity linear treatment pattern 66 were generated with an ultrasound system 100 as described herein.

In one aspect, this disclosure provides an ultrasound system 100 that is capable of depositing ultrasound energy in a treatment pattern 58, the ultrasound energy being deposited in pre-determined amounts with pre-determined spacing along the treatment pattern 58. If the pre-determined amount of ultrasound energy is sufficient to generate a lesion, then a resulting pattern of lesions take the shape of the treatment pattern 58. A user can thereby generate treatment patterns 58 by moving the contact surface 24 along the treatment surface in a desired pattern. The pre-determined amounts of ultrasound energy and the pre-determined spacing can be programmed into the ultrasound system 100, such as by storage in the memory 38. As a user moves the ultrasound probe 10 along the surface, the controller 30 can perform real-time monitoring of the motion or the speed of the ultrasound probe 10 and can direct the ultrasound probe 10 to vary the emission in order to maintain the pre-determined amounts and pre-determined spacing. For example, if the controller 30 senses that the ultrasound probe 10 is moving fast, the controller 30 can instruct the ultrasound probe 10 to emit shorter pulses of ultrasound and to provide less time between pulses. Similarly, if the controller 30 senses that the ultrasound probe 10 is moving slow, the controller 30 can instruct the ultrasound probe 10 to emit longer pulses of ultrasound and to provide more time between pulses. The speed can be measured by the motion sensor 44 or the orientation sensor 46.

In certain aspects, the spacing between pulses is temporal spacing, such that the user interface 28 switch activates one pulse if pressed a single time, or activates a train of equally spaced or non-equally spaced pulses if held.

In one aspect, this disclosure provides an ultrasound system 100 that is capable of guiding a user to move the ultrasound probe 10 at a speed to achieve a pre-determined ultrasound treatment. The pre-determined ultrasound treatment can be programmed into the ultrasound system 100, such as by storage in the memory 38. The ultrasound system 100 can then calculate the required speed of the ultrasound probe 10 along the surface to achieve the pre-determined ultrasound treatment, measure the speed that the ultrasound probe 10 is moving along the treatment surface, and prompt the user to move the ultrasound probe 10 faster if the measured speed is less than the required speed or prompt the user to move the ultrasound probe 10 slower if the measured speed is greater than the required speed. In some aspects, the required speed will be a range of speeds. For example, the optical indicator 54 can provide a visual signal of a first color (for example, red) to prompt the user to move the ultrasound probe 10 slower, a visual signal of a second color (for example, green) to prompt the user to move the ultrasound probe 10 faster, and/or a visual signal of a third color (for example, blue) to indicate to the user that the speed of the ultrasound probe 10 is within a pre-determined acceptable range of speeds. As another example, the audio indicator 50 can provide an audio signal of a first kind (for example, a buzzer sound) to prompt the user to move the ultrasound probe slower, an audio signal of a second kind (for example, a beeping sound) to prompt the user to move the ultrasound probe faster, and/or an audio signal of a third kind (for example, silence or a sound of a bell dinging) to indicate to the user that the speed of the ultrasound probe 10 is within the pre-determined acceptable range of speed. Similarly, as another example, the haptic indicator 52 can provide a haptic signal of a first kind (for example, an intense constant buzzing feeling), a second kind (for example, an intense pulsed buzzing feeling), and/or third kind (for example, no buzzing feeling or a gentle constant or pulsed buzzing feeling) to prompt the user in the same ways described above.

In one aspect, this disclosure provides a system that is capable of delivering constant energy to a target by utilizing real-time sensing of the acoustic coupling to the target. This feature is particularly relevant for embodiments where the ultrasound probe 10 is maneuvered by a user along a surface. In some aspects, the system can stop emission of ultrasound energy if the coupling is interrupted. In some aspects, the system can produce an alarm if the coupling is interrupted. In certain aspects, the ultrasound system 100 can record the position at which the coupling is interrupted, can prompt the user to return to the position, and can resume emission of ultrasound energy once the ultrasound probe 10 has returned to the position and coupling has been re-established.

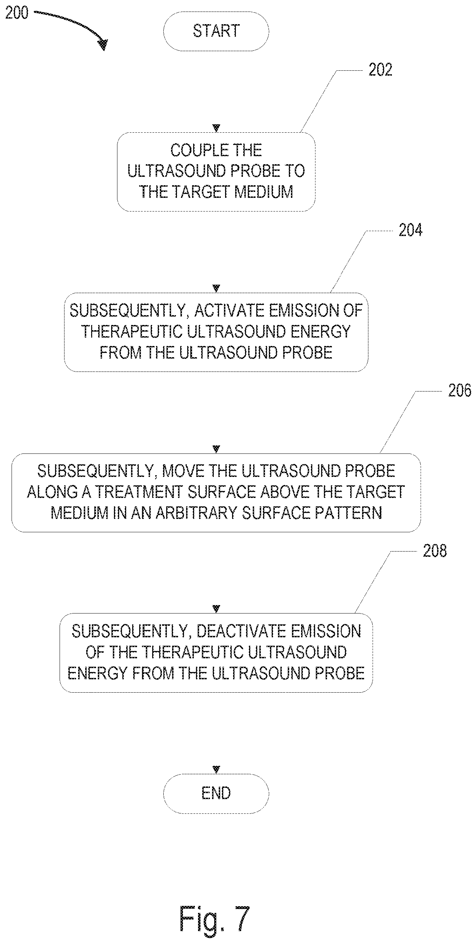

Referring to Fig. 7, this disclosure provides a non-claimed method 200 of depositing therapeutic ultrasound energy. The method 200 can include one or more of the following steps: at process block 202, coupling the ultrasound probe 10 to a target surface; at process block 204, subsequent to the coupling, initiating emission of therapeutic ultrasound energy from the ultrasound probe 10; at process block 206, subsequent to the initiating, moving the ultrasound probe 10 along the target surface such that the contact surface 24 traces a surface pattern on the target surface, thereby directing the therapeutic ultrasound energy beneath the target surface in a treatment pattern 58 that mimics the surface pattern; and at process block 208, subsequent to the moving, deactivating the ultrasound probe 10. In certain aspects, the method can also include directing a secondary energy beneath the target surface, the secondary energy selected from the group consisting of photo-based energy, RF energy, thermal energy, or mechanical energy.

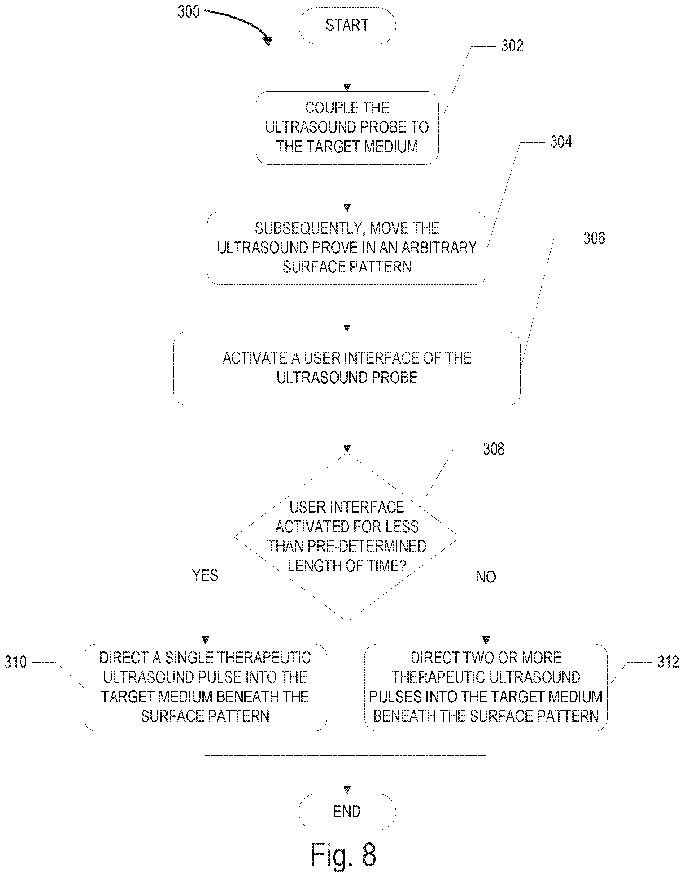

Referring to Fig. 8, this disclosure provide a non-claimed method 300 of depositing therapeutic ultrasound energy from an ultrasound probe into a target medium. The method 300 can include one or more of the following steps: at process block 302, coupling the ultrasound probe to the target medium; at process block 304, suebsequent to the coupling, moving the ultrasound probe along a treatment surface above the target medium in an arbitrary surface pattern; at process block 306, activating a user interface of the ultrasound probe for either less than a pre-determined length of time or greater than or equal to the pre-determined length of time when the ultrasound probe is at a first location; at decision block 308, determining if the user interface is activated for less than a pre-determined length of time or greater than or equal to a pre-determined length of time; at process block 310, if the user interface is activated for less than the pre-determined length of time, directing a single therapeutic ultrasound pulse into the target medium beneath the surface pattern at the first location; and at process block 312, if the user interface is activated for greater than or equal to the pre-determined length of time, directing two or more therapeutic ultrasound pulses into the target medium beneath the surface pattern, the first of the two or more therapeutic ultrasound pulses directed into the target medium at the first location and the second of the two or more therapeutic ultrasound pulses directed into the target medium at a distance from the location along the surface pattern.

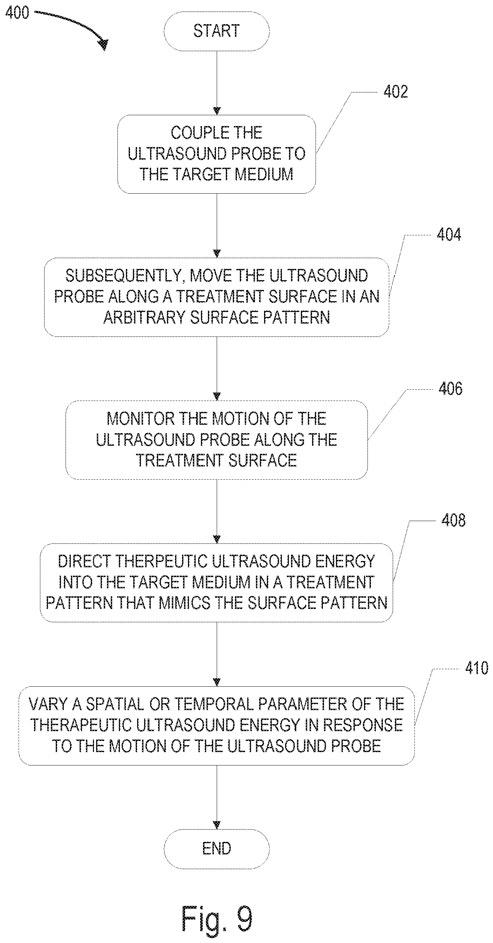

Referring to Fig. 9, this disclosure provides a non-claimed method 400 of depositing therapeutic ultrasound energy from an ultrasound probe into a target medium. The method 400 can include one or more of the following steps: at process block 402, coupling the ultrasound probe to the target medium; at process block 404, subsequent to the coupling, moving the ultrasound probe along a treatment surface above the target medium in an arbitrary surface pattern; at process block 406, monitoring the motion of the ultrasound probe along the treatment surface; at process block 408, directing therapeutic ultrasound energy into the target medium in a treatment pattern beneath the treatment surface that mimics the surface pattern; and at process block 410, varying a spatial or temporal parameter of the therapeutic ultrasound energy in response to the motion of the ultrasound probe.

In certain aspects, the methods 200, 300, 400 can include emitting ultrasound pulses that are spaced apart by a length of time ranging from 1 ms to 1000 ms, including but not limited to, a length of time ranging from 2 ms to 900 ms, 5 ms to 800 ms, 10 ms to 100 ms, 25 ms to 500 ms, 50 ms to 250 ms, 100 ms to 300 ms, 250 ms to 400 ms, or 500 ms to 750 ms. In certain aspects, the methods 200, 300, 400 can include emitting ultrasound energy at a pulse repetition rate of between 0 Hz (continuous wave) and 5 kHz, including but not limited to, between 2 Hz and 1 kHz, between 5 Hz and 100 Hz, or between 10 Hz and 25 Hz.

In certain aspects, the methods 200, 300, 400 can include emitting ultrasound pulses that are spaced apart by distances ranging from 0.1 mm to 10 mm, including but not limited to, distances ranging from 0.2 mm to 8 mm, from 0.5 mm to 5 mm. or from 1 mm to 3 mm. In certain aspects, the method can include emitting continuous ultrasound energy.

In certain aspects, the methods, 200, 300, 400 can include creating lesions spaced apart by distances ranging from 0.1 mm to 10 mm, including but not limited to, distances ranging from 0.2 mm to 8 mm, from 0.5 mm to 5 mm. or from 1 mm to 3 mm. In certain aspects, the method can include lesions that have zero space between them, or a continuous line of lesions.

In certain aspects, the methods 200, 300, 400 can include moving the ultrasound probe 10 a greater distance than the distance between lesions, for example, by taking a non-linear path.

In certain aspects, the methods 200, 300, 400 can determine when and where to emit ultrasound energy and create lesions by measuring the distance that the ultrasound probe 10 travels along whatever path it travels. For example, the ultrasound probe 10 can emit a first ultrasound energy and create a first lesion at a first point, then the ultrasound probe 10 can be moved half of the pre-determined distance in one direction and half of the pre-determined distance in a second direction at a right angle to the first direction, thus resulting in the ultrasound probe 10 having moved to a second point at an absolute distance of $1/\sqrt{2}$ of the pre-determined distance from the first point, then the ultrasound probe 10 can emit a second ultrasound energy and create a second lesion at the second point. This example is non-limiting and serves only to illustrate the aspect of the disclosure where the ultrasound probe 10 can move a pre-determined distance and result in emissions and lesions that are a distance apart that is shorter than the pre-determined distance. In this aspect, the movement of the ultrasound probe 10 determines the distance between emissions and lesions.

In certain aspects, the methods 200, 300, 400 can determine when and where to emit ultrasound energy and create lesions by measuring the absolute distance the ultrasound probe 10 travels in whatever direction it travels. In other words, the methods 200, 300, 400 can map out a circle centered around a first point where the ultrasound probe 10 emits a first ultrasound energy and/or creates a first lesion, then the ultrasound probe 10 can be moved any distance within the circle without emitting further energy and/or creating further lesions, then when the ultrasound probe 10 is moved to any point on the circle the ultrasound probe emits a second ultrasound energy and/or creates a second lesion.

In certain aspects, the methods 200, 300, 400 can determine when and where to emit ultrasound energy and create lesions by measuring the absolute distance the ultrasound probe 10 travels in a pre-determined direction. In other words, the methods 200, 300, 400 can map out a specific point where the next emission and lesion will occur, and if/when the ultrasound probe 10 passes over that specific point, the ultrasound probe can be triggered to emit ultrasound energy and create a lesion.

In certain aspects, the methods 200, 300, 400 can include at least 2, at least 3, at least 4, at least 5, at least 10, at least 20, at least 25, at least 50, at least 100, at least 500, or at least 1000 emissions of pulses of ultrasound energy and/or creations of lesions within a short overall treatment time. The short overall treatment time can be less than 1 hour, one-half hour, 20 minutes, 10 minutes, 1 minute, less than 50 seconds, less than 45 seconds, less than 40 seconds, less than 30 seconds, less than 20 seconds, less than 15 seconds, less than 10 seconds, less than 9 seconds, less than 7 seconds, less than 5 seconds, less than 4 seconds, less than 3 seconds, less than 2 seconds, less than 1 second, less than 0.75 seconds, less than 0.5 seconds, less than 0.25 seconds, less than 0.1 seconds, less than 50 milliseconds, less than 25 milliseconds, less than 10 milliseconds or less than 1 millisecond. Traditional therapeutic treatment systems require overall treatment times that are significantly longer than those afforded by the present disclosure. One advantage of the shortened overall treatment time can be less patient discomfort, among others.

In addition to the fast treatment times and the short overall treatment times, the systems and methods described herein can also afford coverage of large areas in less time that conventional treatment systems.

In certain aspects, the methods 200, 300, 400 can treat at least 10,000 lesions per square centimeter per second, at least 1 million lesions per cubic centimeter per second, at least 100 lesions per square centimeter per second, at least 1 thousand lesions per cubic centimeter per second, at least 50 lesions per square centimeter per second, at least 500 lesions per cubic centimeter per second, at least 25 lesions per cubic centimeter per second, at least 250 lesions per square centimeter per second, at least 10 lesions per square centimeter per second, and at least 100 lesions per cubic centimeter per second.

In certain aspects, the systems and methods described herein can limit a number of ultrasound pulses that are emitted in response to a user action or can limit a number of overall ultrasound pulses for a given treatment. This feature can be utilized for safety by limiting the total energy transmitted to a subject. For example, the systems and methods can limit the emission to 1000 ultrasound pulses of a given energy level, and then once the 1000th pulse is emitted, the controller can terminate emission of the ultrasound energy. This feature can be utilized to define a fixed emission pattern or a fixed energy output.

The systems and methods described herein can provide fast ultrasound lesioning in patients that drastically reduces treatment time and discomfort levels. In certain aspects, the method can include creating lesions at a rate ranging from 0 lesions per second to 1000 lesions per second, including but not limited to, a rate ranging from 1 lesion per second to 900 lesions per second, 2 lesions per second to 800 lesions per second, 10 lesions per second to 500 lesions per second, 50 lesions per second to 100 lesions per second, 100 lesions per second to 700 lesions per second, or 500 lesions per second to 750 lesions per second.

The creation of lesions described herein is achieved by way of delivery of a conformal distribution of ultrasound energy to a target where the lesion is desired. Although the systems and methods of this disclosure are described with respect to the creation of lesions, it should be appreciated that the systems and methods can also be utilized for the creation of other ultrasound effects that can be achieved by delivery of a conformal distribution of ultrasound energy. For example, the systems and methods described herein can create an ablative thermal effect, a non-ablative thermal effect, a nonlinear effect, a biological effect, a stable cavitation effect, an inertial cavitation effect, an acoustic streaming effect, a drug delivery effect, including but not limited to, an effect enhancing the transdermal delivery of a drug, an effect enhancing the efficacy of a drug, and the like, an acousto-mechanical effect, an acousto-elastic effect, a hyperthermia effect, an acoustic resonance effect, a visco-acoustic effect, or any combination thereof. These effects can be provided in an arbitrary pattern as described herein.

The conformal distribution of ultrasound energy can be varied by directing the transducer 12 to emit ultrasound energy having varied spatial and temporal parameters.

The systems and methods described herein can deliver a first conformal distribution of ultrasound energy having a first set of spatial and temporal parameters, a second conformal distribution of ultrasound energy having a second set of spatial and temporal parameters, a third conformal distribution of ultrasound energy having a third set of spatial and temporal parameters, and so on, up to an nth conformal distribution of ultrasound energy having an nth set of spatial and temporal parameters. These conformal distributions can be delivered in an arbitrary pattern as described herein.

The combinations of these conformal distributions of ultrasound energy are too numerous to explicitly recite, so the following list of exemplary combinations are not intended to be limiting.

As one example, a treatment pulse train can provide alternating pulses that have different intensity, have different pulse length, generate different lesion size, and/or have different treatment depths. In a similar fashion, the treatment pulse train can provide periodic rotations of various different pulse properties, such that a first, sixth, eleventh, etc. pulse can have a first set of properties, a second, seventh, twelfth, etc. pulse can have a second set of properties, a third, eighth, thirteenth, etc. pulse can have a third set of properties, a fourth, ninth, fourteenth, etc. pulse can have a fourth set of properties, and a fifth, tenth, fifteenth, etc. pulse can have a fifth set of properties. These periodic rotations of various different pulse properties can repeat

As another example, a treatment pulse train can include a randomized component to determining one or more of the properties described herein. For example, if a pattern of lesions having depths randomized between a minimum depth and a maximum depth is desired, a random depth can be selected for each pulse in real time or prior to treatment and the particular ultrasound pulse can be transmitted to that depth. The same is true for other properties, such as spacing between pulses, either time or distance, pulse intensity, lesion size, and the like.

Treatment pulse trains can be programmed to provide regularly repeating pulses or can be programmed to provide any pattern of treatment pulses. For example, the treatment pulse train can regularly emit a pulse and/or generate a lesion at pre-determined times and/or pre-determined distances. The treatment pulse train can be programmed to provide a given number of pulses that are regularly spaced, either in time or distance, and then "skip" one or more pulses, then resume providing a second given number of pulses, and then "skip" one or more pulses, and so on. The treatment pulse train can be programmed to vary spacing between pulses, in time and/or distance, according to a defined function, such as linear, quadratic, sinusoidal, or any function thought to be useful for treatment. In one specific example, the treatment pulse train can be programmed to deliver pulses that are close to one another immediately following user initiation of the pulse train, but which exponentially space out as time progresses following user initiation of the pulse train.

The various different spatial and/or temporal parameters that can be varied as described above can be prompted by an input to the user interface. Again, the number of ways this aspect can be implemented are too numerous to explicitly recite here, so the following examples are not intended to be limiting. For example, a first press of a switch can initiate a slow pulsed emission with high intensity, then a second press of the switch can initiate a faster pulsed emission with lower intensity, then a third press of the switch can terminate emission. As another example, a first press of a switch can initiate emission at a first depth, a second press of the switch can initiate emission at a second depth, a third press of the switch can initiate emission at a third depth, and so on, until an nth press of the switch terminates emission.

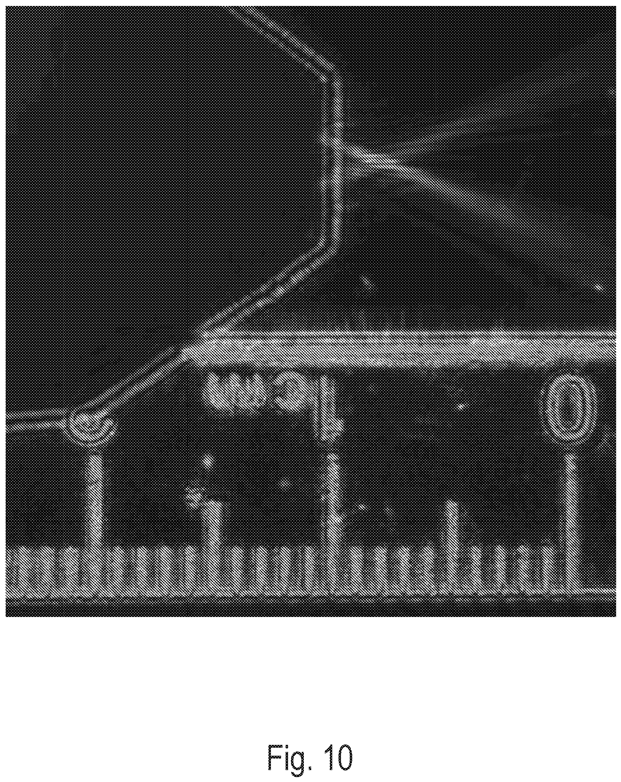

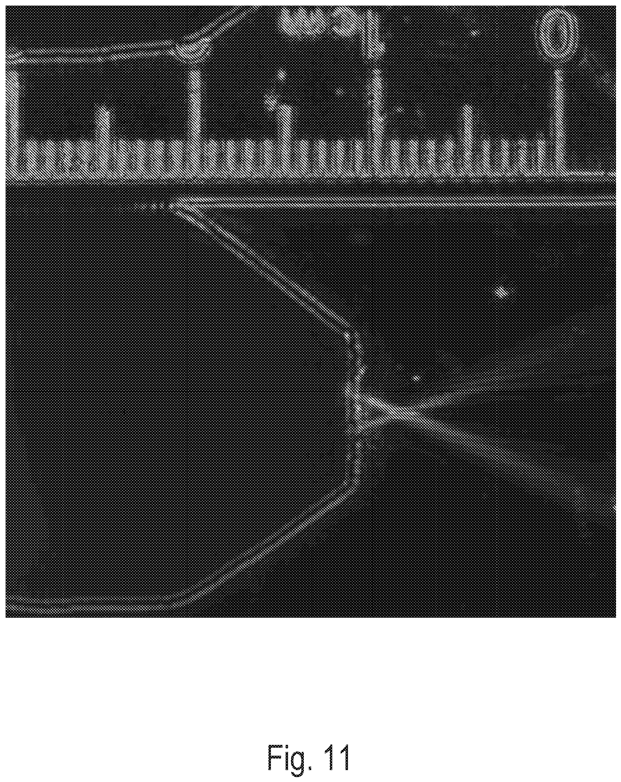

Referring to Fig. 10, a Schlieren image shows the ultrasound emission from an ultrasound probe 10 having a contact surface 24 of a few millimeters and the ultrasound energy focused to a depth of about 2 mm beyond the contact surface 24. Referring to Fig. 11, a Schlieren image shows the ultrasound emission from an ultrasound probe 10 having a contact surface 24 of a few millimeters and the ultrasound energy focused to a depth of about 3 mm beyond the contact surface 24.

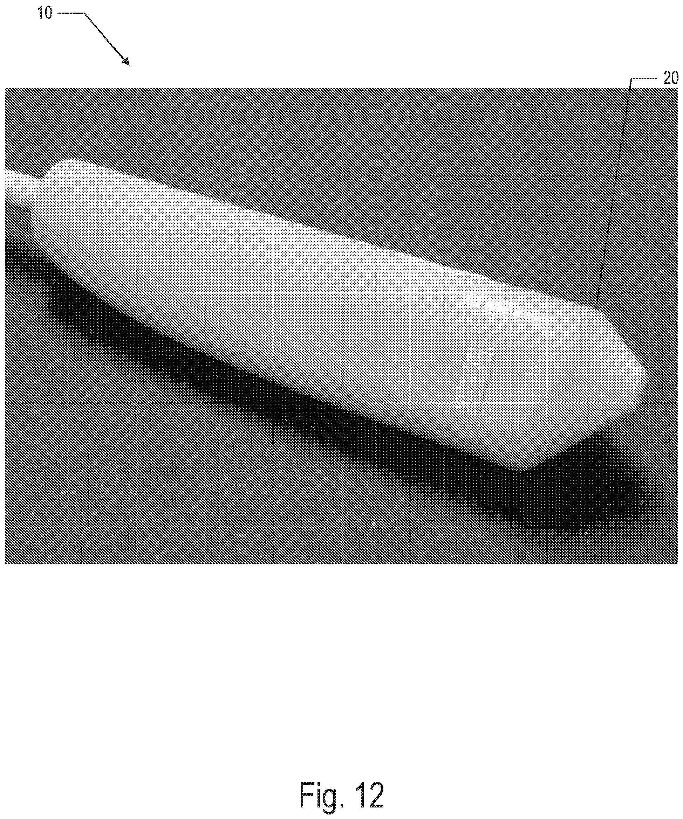

Referring to Fig. 12, an image shows a treatment device 10 according to one aspect of the present disclosure. The image is a perspective view taken from the tip portion 20 end of the treatment device at an angle between 45° and 90° relative to the axial direction 22. The treatment device 10 shown in Fig. 12 also contains the other components which are identified in Fig. 1.

In certain aspects, the ultrasound probe 10 can be configured to interface directly with an unmodified computer or personal device. For example, the ultrasound probe 10 can include all of the electronics necessary for the functions described herein and can be interfaced with the unmodified computer or personal device via a standard interface, such as a USB interface.

In certain aspects, the ultrasound system 100 can include an ultrasound imaging system. The ultrasound imaging system can include an ultrasound imaging transducer or the ultrasound transducer 12 can be a joint therapy/imaging transducer. The ultrasound imaging transducer can be located in the ultrasound probe 10 or can be located in a separate housing. The ultrasound imaging system can include the necessary electronic components to be operable, as would be understood by a person having ordinary skill in the art.

## Example 1. Variable Depth and Size Lesions.

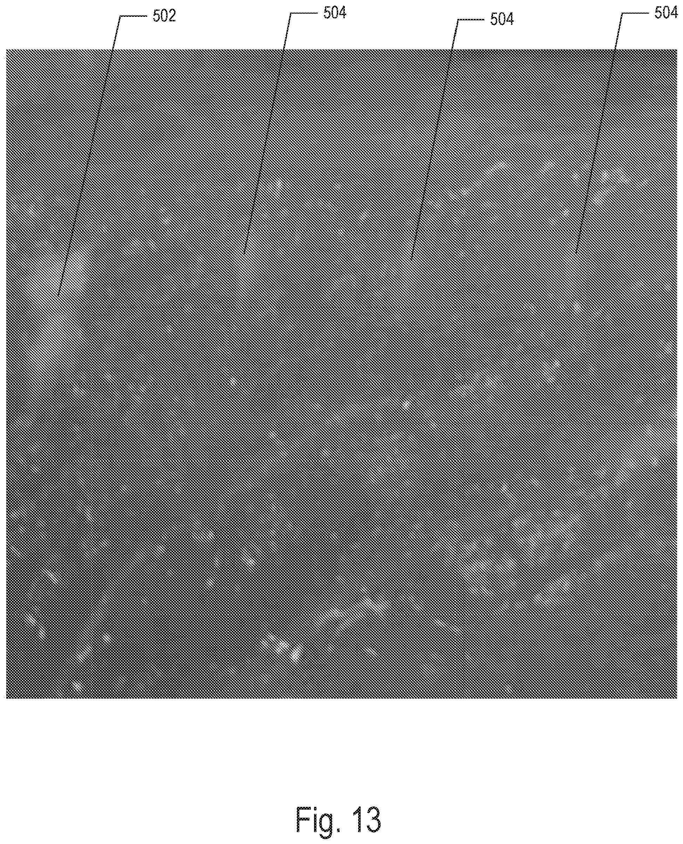

Referring to Fig. 13 a plurality of lesions were created in a line segment pattern by a 7 MHz transducer focused into a porcine muscle tissue, *ex vivo*. Fig. 13 is a photograph of a sagittal plane cross section cut perpendicular to the treatment surface and shows the gross pathology of the ultrasound lesions generated by the treatment. Delivery of a first conformal distribution of ultrasound energy having a first set of spatial and temporal parameters generated one larger lesion 502 at a first depth. Delivery of a second, third, and fourth conformal distribution of ultrasound energy having a second set of spatial and temporal parameters generated three smaller lesions 504 at a second depth. This example exhibits the control of the depth and size of conformal distributions of ultrasound energy and resulting lesions that can be achieved with the methods and systems described herein. Lesions were centered at 3 mm depth. Acoustic energy for the large lesion was 2 J and for the smaller lesions was 0.8 J. The pulse rate was 1 Hz.

## Example 2. Generating Lesions in an Arc Pattern

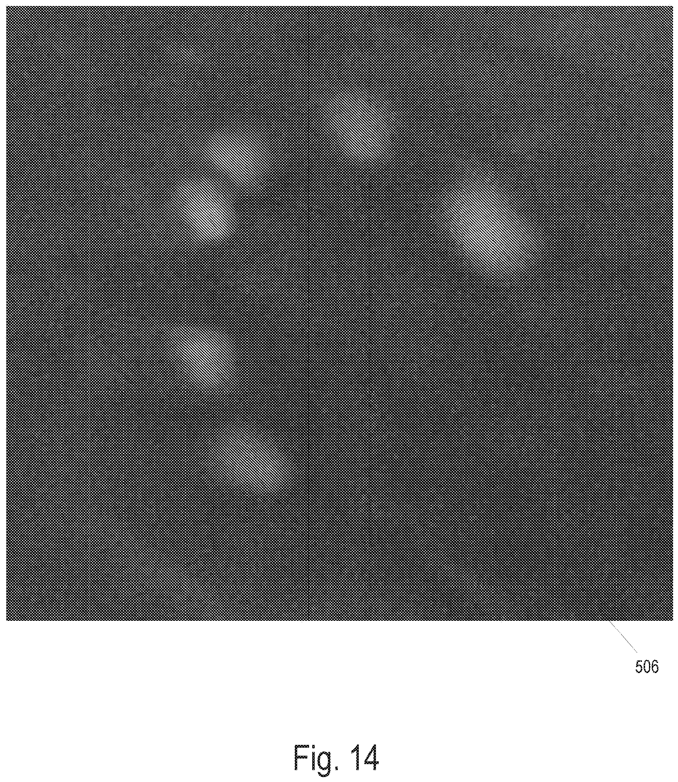

Referring to Fig. 14, a plurality of lesions were created in an arc surface pattern by a 7 MHz transducer focus into a porcine muscle tissue, *ex vivo.* Fig. 14 is a photograph of a coronal plan cross section cut parallel to the treatment surface. Delivery of a first, second, third, fourth, fifth, and sixth conformal distribution of ultrasound energy having the same spatial and temporal parameters generated six lesions 506 at the same depth along an arc treatment pattern beneath the arc surface pattern. This example exhibits the deposition of conformal distributions of ultrasound energy in an arbitrary pattern, in this case, an arc treatment pattern. Lesions were centered at 3 mm depth. The cross section was cut at the same depth of 3 mm. Acoustic energy was 2 J, the pulse rate was 1 Hz, and the arc was approximately 2 cm in diameter.

The present disclosure has been described above with reference to various exemplary configurations. However, those skilled in the art will recognize that changes and modifications may be made to the exemplary configurations without departing from the scope of the present invention. For example, the various operational steps, as well as the components for carrying out the operational steps, may be implemented in alternate ways depending upon the particular application or in consideration of any number of cost functions associated with the operation of the system, e.g., various of the steps may be deleted, modified, or combined with other steps. Further, it should be noted that while the method and system for ultrasound treatment as described above is suitable for use by a user proximate the patient, the system can also be accessed remotely, i.e., the user can view through a remote display having imaging information transmitted in various manners of communication, such as by satellite/wireless or by wired connections such as IP or digital cable networks and the like, and can direct a local practitioner as to the suitable placement for the ultrasound probe. Moreover, while the various exemplary embodiments may comprise non-invasive configurations, system can also be configured for at least some level of invasive treatment application. These and other changes or modifications are intended to be included within the scope of the present invention, which is defined by the independent claim.

## Claims

1. An ultrasound treatment system comprising:

   an ultrasound probe (10) having a contact surface (24), the ultrasound probe movable by a user along a treatment surface in an arbitrary surface pattern (58) while maintaining contact between the contact surface and the treatment surface;
   a control module (30) operably coupled to the ultrasound probe, the control module, in use, controlling the emission of a therapeutic ultrasound energy from the ultrasound probe;
   a position sensor (44, 46) that, in use, measures a position of the ultrasound probe along the treatment surface and transmits a position signal to the control module corresponding to the position of the ultrasound probe along the treatment surface; and
   a power supply (28) that, in use, provides power to the ultrasound probe sufficient for the emission of the therapeutic ultrasound energy,

   wherein the control module, in response to the position signal indicating that the ultrasound probe is moving along the treatment surface, and in response to the measured position, varies a pulse width of the therapeutic ultrasound energy, a power amplitude of the therapeutic ultrasound energy, and a timing between pulses of the therapeutic ultrasound energy to produce a series of ultrasound pulses that have equal energy and are equally spaced along the arbitrary surface pattern.

2. The ultrasound treatment system of claim 1, wherein the contact surface has a maximum physical dimension of less than 2 cm.

3. The ultrasound treatment system of claim 1, the ultrasound probe comprising a transducer (12) that is stationary relative to the contact surface.

4. The ultrasound treatment system of claim 1, the ultrasound probe further comprising a coupling sensor (48) configured to sense an acoustic coupling between the ultrasound probe and the treatment surface and communicate a coupling status signal to the control module that is indicative of a coupling status between the ultrasound probe and the treatment surface.

5. The ultrasound treatment system of claim 1, wherein the ultrasound probe is configured to emit at least five pulses of ultrasound energy in less than 1 second.

6. The ultrasound treatment system of claim 1, wherein the ultrasound probe is configured to emit the ultrasound energy at a pulse repetition rate of between 0 Hz and 5 kHz.

7. The ultrasound treatment system of claim 1, wherein the series of ultrasound pulses produce lesions that are equal in size and relative spacing.

## Patentansprüche

1. Ein Ultraschallbehandlungssystem, aufweisend:

   eine Ultraschallsonde (10) mit einer Kontaktfläche (24), wobei die Ultraschallsonde von einem Benutzer entlang einer Behandlungsfläche in einem beliebigen Oberflächenmuster (58) bewegt werden kann, während der Kontakt zwischen der Kontaktfläche und der Behandlungsfläche aufrechterhalten wird;
   ein Steuermodul (30), das betriebsfähig mit der Ultraschallsonde gekoppelt ist, wobei das Steuermodul im Gebrauch die Emission einer therapeutischen Ultraschallenergie von der Ultraschallsonde steuert;
   einen Positionssensor (44, 46), der bei der Verwendung eine Position der Ultraschallsonde entlang der Behandlungsoberfläche misst und ein Positionssignal an das Steuermodul überträgt, das der Position der Ultraschallsonde entlang der Behandlungsoberfläche entspricht; und
   eine Stromversorgung (28), die im Gebrauch die Ultraschallsonde mit Energie versorgt, die für die Emission der therapeutischen Ultraschallenergie ausreicht,
   wobei das Steuermodul in Reaktion auf das Positionssignal, das anzeigt, dass sich die Ultraschallsonde entlang der Behandlungsoberfläche bewegt, und in Reaktion auf die gemessene Position eine Impulsbreite der therapeutischen Ultraschallenergie, eine Leistungsamplitude der therapeutischen Ultraschallenergie und ein Timing zwischen den Impulsen der therapeutischen Ultraschallenergie variiert, um eine Serie von Ultraschallimpulsen zu erzeugen, die die gleiche Energie haben und entlang des beliebigen Oberflächenmusters gleich beabstandet sind.

**2.** Ultraschallbehandlungssystem nach Anspruch 1, wobei die Kontaktfläche eine maximale physische Abmessung von weniger als 2 cm aufweist.

**3.** Ultraschallbehandlungssystem nach Anspruch 1, wobei die Ultraschallsonde einen relativ zur Kontaktfläche feststehenden Energieumwandler (12) aufweist.

**4.** Ultraschallbehandlungssystem nach Anspruch 1, wobei die Ultraschallsonde ferner einen Kopplungssensor (48) aufweist, der konfiguriert ist, um eine akustische Kopplung zwischen der Ultraschallsonde und der Behandlungsoberfläche zu erfassen und ein Kopplungsstatussignal an das Steuermodul zu übermitteln, das einen Kopplungsstatus zwischen der Ultraschallsonde und der Behandlungsoberfläche anzeigt.

**5.** Ultraschallbehandlungssystem nach Anspruch 1, wobei die Ultraschallsonde ist, um mindestens fünf Ultraschallimpulse in weniger als einer Sekunde auszusenden.

**6.** Ultraschallbehandlungssystem nach Anspruch 1, wobei die Ultraschallsonde konfiguriert, um die Ultraschallenergie mit einer Impulswiederholungsrate zwischen 0 Hz und 5 kHz auszusenden.

**7.** Ultraschallbehandlungssystem nach Anspruch 1, wobei die Serie von Ultraschallimpulsen Läsionen erzeugt, die in Größe und relativem Abstand gleich sind.

**Revendications**

**1.** Système de traitement par ultrasons comprenant :

une sonde à ultrasons (10) présentant une surface de contact (24), la sonde à ultrasons pouvant être déplacée par un utilisateur le long d'une surface de traitement selon un motif de surface arbitraire (58) tout en maintenant le contact entre la surface de contact et la surface de traitement ;
un module de commande (30) couplé de manière fonctionnelle à la sonde à ultrasons, le module de commande, lors de l'utilisation, commandant l'émission d'une énergie ultrasonore thérapeutique depuis la sonde à ultrasons ;
un capteur de position (44, 46) qui, lors de l'utilisation, mesure une position de la sonde à ultrasons le long de la surface de traitement et transmet au module de commande un signal de position correspondant à la position de la sonde à ultrasons le long de la surface de traitement ; et
une alimentation électrique (28) qui, lors de l'utilisation, fournit à la sonde à ultrasons de l'énergie suffisante pour l'émission de l'énergie ultrasonore thérapeutique,
dans lequel le module de commande, en réponse au signal de position indiquant que la sonde à ultrasons se déplace le long de la surface de traitement, et en réponse à la position mesurée, varie la largeur d'impulsions de l'énergie ultrasonore thérapeutique, l'amplitude de puissance de l'énergie ultrasonore thérapeutique et le temps entre les impulsions de l'énergie ultrasonore thérapeutique afin de produire une série d'impulsions ultrasonores qui présentent une énergie égale et qui sont espacées de manière égale le long du motif de surface arbitraire.

**2.** Système de traitement par ultrasons selon la revendication 1, dans lequel la surface de contact présente une dimension physique maximale inférieure à 2 cm.

**3.** Système de traitement par ultrasons selon la revendication 1, la sonde à ultrasons comprenant un transducteur (12) qui est stationnaire par rapport à la surface de contact.

**4.** Système de traitement par ultrasons selon la revendication 1, la sonde à ultrasons comprenant en outre un capteur de couplage (48) conçu pour détecter un couplage acoustique entre la sonde à ultrasons et la surface de traitement et communiquer au module de commande un signal d'état de couplage, qui indique un état de couplage entre la sonde à ultrasons et la surface de traitement.

**5.** Système de traitement par ultrasons selon la revendication 1, dans lequel la sonde à ultrasons est conçue pour émettre au moins cinq impulsions d'énergie ultrasonore en moins d'une seconde.

**6.** Système de traitement par ultrasons selon la revendication 1, dans lequel la sonde à ultrasons est conçue pour émettre l'énergie ultrasonore à une fréquence de répétition d'impulsions comprise entre 0 Hz et 5 kHz.

**7.** Système de traitement par ultrasons selon la revendication 1, dans lequel la série d'impulsions ultrasonores produit des lésions qui sont égales en taille et en espacement relatif.

Fig. 1

100

ULTRASOUND
PROBE
10

POWER SOURCE
56

CONTROL
MODULE
30

DISPLAY
32

ELECTRICAL
SENSOR
42

AUDIO
INDICATOR
50

REMOTE USER
INTERFACE
34

MOTION SENSOR
44

HAPTIC
INDICATOR
52

USER TERMINAL
36

ORIENTATION
SENSOR
46

OPTICAL
INDICATOR
54

MEMORY
38

COUPLING
SENSOR
48

SECONDARY ENERGY
DELIVERY MODULES
40

Fig. 2

Fig. 3

Fig. 4

Fig. 5

Fig. 6

200

START

202

COUPLE THE
ULTRASOUND PROBE TO
THE TARGET MEDIUM

204

SUBSEQUENTLY, ACTIVATE EMISSION OF
THERAPEUTIC ULTRASOUND ENERGY
FROM THE ULTRASOUND PROBE

206

SUBSEQUENTLY, MOVE THE ULTRASOUND PROBE
ALONG A TREATMENT SURFACE ABOVE THE TARGET
MEDIUM IN AN ARBITRARY SURFACE PATTERN

208

SUBSEQUENTLY, DEACTIVATE EMISSION
OF THE THERAPEUTIC ULTRASOUND
ENERGY FROM THE ULTRASOUND PROBE

END

Fig. 7

Fig. 8

400

START

402

COUPLE THE
ULTRASOUND PROBE TO
THE TARGET MEDIUM

404

SUBSEQUENTLY, MOVE THE ULTRASOUND
PROBE ALONG A TREATMENT SURFACE IN AN
ARBITRARY SURFACE PATTERN

406

MONITOR THE MOTION OF THE
ULTRASOUND PROBE ALONG THE
TREATMENT SURFACE

408

DIRECT THERPEUTIC ULTRASOUND ENERGY
INTO THE TARGET MEDIUM IN A TREATMENT
PATTERN THAT MIMICS THE SURFACE PATTERN

410

VARY A SPATIAL OR TEMPORAL PARAMETER OF THE
THERAPEUTIC ULTRASOUND ENERGY IN RESPONSE
TO THE MOTION OF THE ULTRASOUND PROBE

END

Fig. 9

Fig. 10

Fig. 11

Fig. 12

Fig. 13

506

Fig. 14

**EP 3 280 495 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012165668 A
- US 2015088182 A
- US 2006122509 A
- US 547012